# EUROPEAN PATENT SPECIFICATION

(11) **EP 3 196 271 B1**
(45) Date of publication and mention of the grant of the patent: **09.01.2019**
(21) Application number: 16783059.5
(22) Date of filing: 12.04.2016
(51) Int. Cl.: C09K 3/00, C06B 43/00, C06D 5/00, C07C 281/00, C08J 9/10, C08K 5/24, C08L 101/00, C08J 9/06, C08J 9/08, C07C 281/08

(54) **GAS-GENERATING AGENT, AND PROCESS FOR PRODUCING FOAMED OBJECT USING SAME**
GASERZEUGENDES MITTEL UND VERFAHREN ZUR HERSTELLUNG EINES GESCHÄUMTEN OBJEKTS UNTER VERWENDUNG DAVON
AGENT GÉNÉRATEUR DE GAZ, ET PROCÉDÉ DE PRODUCTION D'UN OBJET ALVÉOLAIRE À L'AIDE DE CELUI-CI

(30) Priority: 23.04.2015 JP 2015088659
(43) Date of publication of application: 26.07.2017
(73) Proprietor: Mitsubishi Gas Chemical Company, Inc., Tokyo 100-8324 (JP); Eiwa Chemical Ind. Co., Ltd., Kyoto-shi, Kyoto 604-8161 (JP)
(72) Inventor: SAKAI, Haruka, Tokyo 125-8601 (JP); KAGEYAMA, Takuya, Tokyo 125-8601 (JP); UERA, Kazuyoshi, Tokyo 125-8601 (JP); IWATA, Tomoki, Yamakita-machi Ashigarakami-gun Kanagawa 258-0112 (JP); YASUDA, Yuji, Handa-shi Aichi 475-0033 (JP); NAKATA, Kazuya, Handa-shi Aichi 475-0033 (JP); IWASAKI, Hiroshi, Handa-shi Aichi 475-0033 (JP); KASUGA, Mitsuru, Handa-shi Aichi 475-0033 (JP)
(74) Representative: Müller-Boré & Partner Patentanwälte PartG mbB
(86) International application number: PCT/JP2016/061805
(87) International publication number: WO 2016/171038

(56) References cited:
- CN-A- 101 341 199
- JP-A- H10 509 455
- JP-A- 2000 511 177
- JP-A- 2001 139 928
- JP-A- 2003 501 419
- VAILLANCOURT, VALERIE A. ET AL.: 'Synthesis and biological activity of aminoguanidine and diaminoguanidine analoques of the antidiabetic/ antiobesity agent 3-guanidinopropionic acid' JOURNAL OF MEDICINAL CHEMISTRY vol. 44, no. 8, 2001, ISSN 0022-2623 pages 1231 - 1248, XP002728737
- CLIVE, DERRICK L. J. ET AL.: 'Preparation of ?- (2,2-Diphenylhydrazino)lactones and Related Compounds by Radical Cyclization: Use of Glyoxylic Acid Hydrazone Derivatives' JOURNAL OF ORGANIC CHEMISTRY vol. 66, no. 4, 2001, ISSN 0022-3263 pages 1233 - 1241, XP055324465

## Description

### Technical Field

The present invention relates to a gas generating agent and a method for producing a foam of a thermoplastic resin, a rubber, using the gas generating agent.

### Background Art

Azodicarbonamide (hereinafter also referred to as "ADCA"), which is a typical gas generating agent, is a compound used in a broad range of applications as a chemical blowing agent for plastics and rubbers in general. Advantages of ADCA include that the amount of generated gas is 200 mL/g or more and is larger than that of other commercially available chemical blowing agents, that the degradation starting temperature can be lowered to near 140°C by using a blowing auxiliary in combination although ADCA when used alone has a relatively high degradation starting temperature of 200 to 210°C so that ADCA is usable as a chemical blowing agent for general-purpose plastics (for example, thermoplastic resins) and rubbers, and that the generated gas is mainly composed of nitrogen and is self-extinguishing so that ADCA is highly safe in terms of handling (see, for example, Patent Literature 1 and Non Patent Literature 1).

On the other hand, disadvantages of ADCA include that a small amount of ammonia gas is contained in the gas generated during foaming and this ammonia gas is corrosive, and that cyanic acid, which is a degradation product of ADCA, is sublimable and polymerizes after sublimation to become corrosive cyanuric acid, which is likely to cause metal mold contamination. In the fields that do not welcome such disadvantages of ADCA, i.e., corrosion by ammonia and metal mold contamination by a degradation product, 4,4'-oxybis(benzenesulfonyl hydrazide) (hereinafter also referred to as "OBSH") is used as a gas generating agent, the generated gases of which are only nitrogen and water and also the degradation product of which is not contaminating. OBSH has a low degradation starting temperature of about 170°C and is mainly used for ethylene-propylene-diene rubber (EPDM) for weather strips and chloroprene rubber (CR) for wet suits.

In addition to ADCA and OBSH described above, hydrazine derivatives such as semicarbazide, carbohydrazide, and carbazic acid esters can also be used as gas generating agents. These hydrazine derivatives are often used in applications such as crosslinking agents and curing agents for synthetic resins, pharmaceutical and agrochemical raw materials and intermediates, and reducing agents (oxygen scavengers) for boilers. In gas generating agent applications, for example, semicarbazide and carbohydrazide are known to form complexes with manganese nitrate and thus become gas generating agents for air bags (see, for example, Patent Literatures 2, 3, and 4).

Patent Literature 5 discloses that a reaction product obtained by reacting a compound having an -NH₂ group, such as semicarbazide or carbohydrazide, with an organic compound having a -CHO group in the structural formula or an organic compound capable of forming a -CHO group is used as a gas generating component and can be used as a gas generating agent in combination with an oxidizer.

### Citation List

### Patent Literature

Patent Literature 1: Japanese Patent Laid-Open No. 11-21364
Patent Literature 2: International Publication No. WO 98/29362
Patent Literature 3: International Publication No. WO 98/29425
Patent Literature 4: International Publication No. WO 98/29426
Patent Literature 5: International Publication No. WO 96/10000 Patent Literature 6: CN-A-101 341 199

### Non Patent Literature

Non Patent Literature 1: Hitoshi Kondo, "The Characteristic of Foaming Agents", Nippon Gomu Kyokaishi, The Society of Rubber Science and Technology, Japan, 2001, Vol. 74, No. 10, pp. 406-411

### Summary of the Invention

### Technical Problem

As a gas generating agent, a compound is desired that, for example, has a degradation starting temperature at which the compound is usable as a chemical blowing agent for general-purpose plastics (for example, thermoplastic resins) and rubbers, generates a large amount of gas, barely contains corrosive gas such as ammonia in the generated gas, and is usable used alone.

However, the ADCA has problems such as corrosion by ammonia in the generated gas and metal mold contamination by a degradation product as described above (see, for example, Patent Literature 1 and Non Patent Literature 1).

On the other hand, the OBSH has a low degradation starting temperature and does not result in metal mold contamination by a degradation product because the generated gases contain only nitrogen and water. However, the OBSH is problematic from the viewpoint of economy and resource saving because the amount of generated gas is as small as 120 mL/g and it is necessary to increase the amount of OBSH added to obtain a desired expansion ratio (see, for example, Non Patent Literature 1).

Hydrazine derivatives such as semicarbazide, carbohydrazide, and carbazic acid esters are easily synthesized and are widely used compounds. However, the amount of generated gas is small when semicarbazide and carbohydrazide are used alone or in a hydrochloride form as chemical blowing agents for general-purpose plastics (for example, thermoplastic resins) and rubbers. The degradation starting temperature of methyl carbazate is lower than 100°C, and the degradation starting temperature is too low to use methyl carbazate as the above chemical blowing agent. That is to say, when compared with ADCA and OBSH, these hydrazine derivatives have problems, for example, in that the amount of generated gas is small or that the gas generating temperature is greatly different from the molding temperatures of general-purpose plastics (for example, thermoplastic resins) and rubbers.

The reaction product disclosed in Patent Literature 5, which is obtained by reacting a compound having an - NH₂ group, such as semicarbazide or carbohydrazide, with an organic compound having a -CHO group in the structural formula or an organic compound capable of forming a -CHO group, needs to be used in combination with an oxidizer. Patent Literature 6 discloses a gas generating agent comprising p-toluene sulfonylsemicarbazide.

The present invention has been conceived in view of the above problems, and an object thereof is to provide a gas generating agent comprising a compound, a raw material of which is an easily synthesized and widely used hydrazine derivative and which, for example, has a degradation starting temperature at which the compound is usable as a chemical blowing agent for general-purpose plastics (for example, thermoplastic resins) and rubbers, generates a large amount of gas, barely contains corrosive gas such as ammonia in the generated gas, and is usable as a gas generating agent even used alone. Solution to the Problem

As a result of having conducted diligent research on the above problems, the inventors found that a compound represented by a specific chemical formula, such as a compound obtained by subjecting compound (A) having a hydrazide group represented by -C(=O)NHNH₂ within the molecule such as semicarbazide, carbohydrazide or a carbazic acid ester, and glyoxylic acid (B) to a dehydrative condensation reaction, solves the above problems, and the inventors accomplished the present invention.

That is to say, the present invention is as follows.
[1] A gas generating agent comprising a compound represented by general formula (1) below. 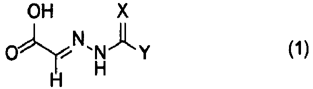 wherein X is an oxygen atom or an imino group, and Y is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (2) below. 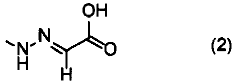
[2] The gas generating agent according to [1], wherein the compound represented by general formula (1) comprises a structure derived from compound (A) represented by general formula (3) below and a structure derived from glyoxylic acid (B). 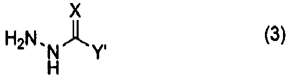 wherein X is an oxygen atom or an imino group, and Y' is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (4) below. 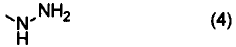
[3] The gas generating agent according to [1], wherein the compound represented by general formula (1) is a compound represented by formula (5) below. 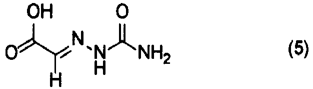
[4] The gas generating agent according to [1], wherein the compound represented by general formula (1) is a compound represented by formula (6) below. 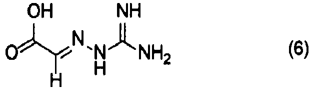
[5] The gas generating agent according to [1], wherein the compound represented by general formula (1) is a compound represented by general formula (7) below. 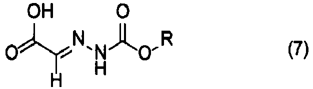 wherein R is a hydrogen atom, an alkyl group having 1 to 8 carbon atoms, an allyl group having 3 to 8 carbon atoms, a benzyl group, an alkenyl group having 2 to 8 carbon atoms, or an aryl group having 6 to 10 carbon atoms.
[6] The gas generating agent according to [1], wherein the compound represented by general formula (1) is a compound represented by formula (8) below. 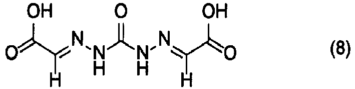
[7] The gas generating agent according to any of [1] to [6], wherein the gas generating agent is a microcapsule-based agent.
[8] A foamable composition comprising the gas generating agent according to any of [1] to [7] and a material to be foamed.
[9] The foamable composition according to [8], wherein the material to be foamed is a thermoplastic resin and/or a rubber.
[10] A method for producing a foam, comprising a step of heating the foamable composition according to [8] or [9].
[11] A foam, wherein the foam is obtained by foaming the foamable composition according to [8] or [9].
[12] A compound represented by formula (8) below.

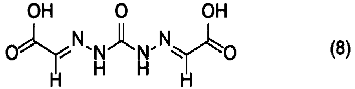

### Advantageous Effect of the Invention

The present invention can provide a gas generating agent comprising a compound, raw materials of which are an easily synthesized and widely used chemical raw material hydrazine derivative and glyoxylic acid and which, for example, has a degradation starting temperature at which the compound is usable as a chemical blowing agent for general-purpose plastics (for example, thermoplastic resins) and rubbers, generates a large amount of gas, barely contains corrosive gas such as ammonia in the generated gas, and is usable as a gas generating agent even used alone.

### Description of Embodiment

Below, an embodiment (hereinafter also referred to as "the present embodiment") of the present invention is described in detail. The following embodiment is an example for describing the present invention, and the present invention is not limited only to the embodiment.

The gas generating agent of the present embodiment comprises a compound represented by general formula (1) below. 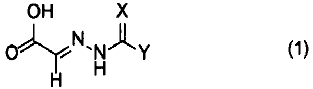 (In formula (1), X is an oxygen atom or an imino group, and Y is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (2) below.)

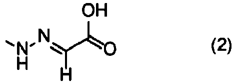

The compound represented by general formula (1) generates a large amount of gas, barely contains corrosive gas such as ammonia in the generated gas, and can be suitably used as a gas generating agent even used alone. Although the mechanism with which the compound represented by general formula (1) provides such effects is not clear, the present inventors infer that it is due to the generation of nitrogen gas from the hydrazide structure and the generation of carbon dioxide gas from the carboxy group in the compound represented by general formula (1).

The amount of generated gas during the foaming of the gas generating agent of the present embodiment is preferably 120 mL/g or more.

The amount of generated ammonia during the foaming of the gas generating agent of the present embodiment is preferably 10 mg/g or less, more preferably 5 mg/g or less, and even more preferably 2 mg/g or less.

The degradation starting temperature of the gas generating agent of the present embodiment is preferably a degradation starting temperature at which the gas generating agent is usable as a chemical blowing agent for, for example, general-purpose plastics (for example, thermoplastic resins) and rubbers and, specifically, is preferably 90 to 380°C and more preferably 110 to 250°C.

In the present embodiment, the amount of generated gas during foaming, the amount of generated ammonia, and the degradation starting temperature of the gas generating agent can be measured by the methods described in the Examples below.

The compound represented by general formula (1) is preferably a compound obtained by subjecting compound (A) represented by general formula (3) below and glyoxylic acid (B) to a dehydrative condensation reaction.

Accordingly, the compound represented by general formula (1) preferably includes a structure derived from compound (A) represented by general formula (3) below and a structure derived from glyoxylic acid (B). 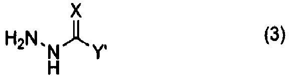 (In formula (3), X is an oxygen atom or an imino group, and Y' is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (4) below.)

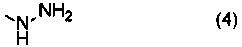

In the compound represented by general formula (1), X is preferably an oxygen atom. In the compound represented by general formula (1), Y is preferably an amino group or an alkoxy group having 1 to 4 carbon atoms.

More preferably, the gas generating agent of the present embodiment comprises a compound obtained by subjecting compound (A-1) having a hydrazide group represented by -C(=O)NHNH₂ within the molecule and glyoxylic acid (B) to a dehydrative condensation reaction. In the description below, compound (A-1) having a hydrazide group represented by -C(=O)NHNH₂ within the molecule may be simply referred to as compound (A-1) having a hydrazide group. The compound obtained by subjecting compound (A-1) having a hydrazide group and glyoxylic acid (B) to a dehydrative condensation reaction is a compound that has a degradation starting temperature at which the compound is usable as a chemical blowing agent for general-purpose plastics (for example, thermoplastic resins) and rubbers, generates a large amount of gas, barely contains corrosive gas such as ammonia in the generated gas, and is usable as a gas generating agent used alone.

Next, compound (A-1) having a hydrazide group represented by -C(=O)NHNH₂ within the molecule is described, which serves as a raw material of a compound usable as the gas generating agent of the present embodiment. The kind of compound (A-1) having a hydrazide group is not particularly limited, and examples include acetic acid hydrazide, propionic acid hydrazide, butyric acid hydrazide, acrylic acid hydrazide, methacrylic acid hydrazide, crotonic acid hydrazide, benzoic acid hydrazide, 4-methylbenzoic acid hydrazide, phenylacetic acid hydrazide, oxamic acid hydrazide, oxalyldihydrazide, octanic acid hydrazide, adipic acid dihydrazide, cyanoacetic acid dihydrazide, succinic acid dihydrazide, azelaic acid dihydrazide, sebacic acid dihydrazide, terephthalic acid dihydrazide, isophthalic acid dihydrazide, 1-naphthohydrazide, 4-aminobenzoic acid hydrazide, 4-hydroxybenzoic acid hydrazide, 4-methoxybenzoic acid hydrazide, 3-methoxybenzoic acid hydrazide, 4-nitrobenzoic acid hydrazide, 3-nitrobenzoic acid hydrazide, salicylic acid hydrazide, 3-hydroxy-2-naphthoic acid hydrazide, isonicotinic acid hydrazide, nicotinic acid hydrazide, semicarbazide, carbohydrazide, salts (hydrochloric acid salts, sulfuric acid salts, nitric acid salts) of these hydrazides, and carbazic acid esters such as methyl carbazate, ethyl carbazate, and t-butyl carbazate.

In the present embodiment, one of these may be used singly, or two or more may be used in combination. Preferable among these are semicarbazide and carbohydrazide that are compounds in which a nitrogen atom is bonded to the above hydrazide group, hydrochloric acid salts thereof, and/or carbazic acid esters such as methyl carbazate, ethyl carbazate, and t-butyl carbazate that are compounds in which an oxygen atom is bonded to the hydrazide group. A compound obtained by a condensation reaction between such compound (A-1) having a hydrazide group and glyoxylic acid (B) has a degradation starting temperature at which the compound is usable as a chemical blowing agent for general-purpose plastics (for example, thermoplastic resins) and rubbers, generates a large amount of gas, and is thus preferable. The aforementioned compounds having a hydrazide group are all known compounds and are obtainable as commercially available products.

Another raw material of the compound usable as the gas generating agent of the present embodiment is glyoxylic acid (B). Glyoxylic acid is a known compound, and a commercially available product can be used.

As the gas generating agent of the present embodiment, a compound represented by formula (5), formula (8), or general formula (7) below is preferable because the compound has a degradation starting temperature at which the compound is usable as a chemical blowing agent for general-purpose plastics (for example, thermoplastic resins) and rubbers, the amount of generated gas is large, and the amount of generated ammonia is small. 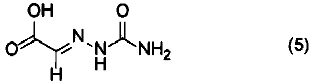 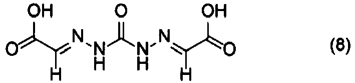 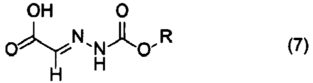 (In formula (7), R is a hydrogen atom, an alkyl group having 1 to 8 carbon atoms, an allyl group having 3 to 8 carbon atoms, a benzyl group, an alkenyl group having 2 to 8 carbon atoms, or an aryl group having 6 to 10 carbon atoms.)

A compound represented by formula (6) is preferable as the gas generating agent of the present embodiment.

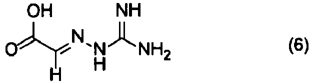

Next, the conditions of a dehydrative condensation reaction between compound (A-1) having a hydrazide group represented by -C(=O)NHNH₂ within the molecule and glyoxylic acid (B) is described in detail. The reaction conditions are not particularly limited, and, for example, the reaction can be carried out by using 1 mol to an excess of glyoxylic acid (B) and as necessary 0.001 to 1 mol of an acid catalyst as a condensation promoter per mole of compound (A-1) having a hydrazide group, and performing stirring in a polar solvent such as water or alcohol under normal pressure at 0 to 100°C for 10 minutes to 24 hours. After reaction, the intended product can be separated and purified by a known method. An example of a separation and purification method is a method in which crystals are obtained by cooling the reaction solution by ice water to precipitate and isolate crystals.

In the dehydrative condensation reaction, the proportion of the compound (A-1) having a hydrazide group to glyoxylic acid (B) used is not particularly limited, and is preferably 1:1 to 1:100 and particularly preferably 1:1 to 1:10 as a molar ratio. The reaction may be performed at room temperature, or may be performed with increasing temperature as necessary. In consideration of the boiling point of raw-material glyoxylic acid (B) being 111°C, the reaction temperature is preferably 0 to 100°C and particularly preferably 20 to 80°C.

The polar solvent that can be used in the reaction is not particularly limited, and examples include water, methanol, ethanol, propanol, isopropyl alcohol, butanol, and isobutyl alcohol. In particular, water is preferable with which the purification of the product is easy and which is highly economical.

The condensation promoter is not particularly limited, and examples include hydrochloric acid, sulfuric acid, acetic acid, p-toluenesulfonic acid, nitric acid, oxalic acid, phosphoric acid, hydrobromic acid, hydroiodic acid, sulfamic acid, and perchloric acid. Among these, hydrochloric acid and sulfuric acid are preferable in terms of economy.

After the end of reaction, crystals precipitated by a cooling operation are filtered off, washed with water, alcohol, and then dried under reduced pressure, and it is thereby possible to obtain a compound used as the gas generating agent of the present embodiment (a compound obtained by a dehydrative condensation reaction between the compound (A-1) having a hydrazide group represented by -C(=O)NHNH₂ within the molecule and glyoxylic acid (B)). The compound used as the gas generating agent may be in the form of a hydrate, or may be in the form of a salt formed with an acid used as a condensation promoter.

The gas generating agent comprising the compound thus obtained can be suitably used as a chemical blowing agent for general-purpose plastics (for example, thermoplastic resins) and rubbers. Preferably, the compound used for the gas generating agent of the present embodiment can suppress the generation of corrosive gas such as ammonia and provide a foam having a high expansion ratio when used alone at the foam molding temperature (for example, about 130 to 250°C) of general-purpose plastics (for example, thermoplastic resins) and rubbers.

Although the gas generating agent of the present embodiment can be suitably used as a chemical blowing agent for general-purpose plastics (for example, thermoplastic resins) and rubbers even when composed solely of the compound represented by general formula (1), the gas generating agent may further comprise an adjuvant in addition to the compound represented by general formula (1). The adjuvant is not particularly limited, and examples include oxidizers, crosslinking agents (C), nitrous acid salts, and hydrotalcite. When the gas generating agent of the present embodiment further comprises such an adjuvant, there is a tendency that the generation of corrosive gas such as ammonia can be further suppressed, and the temperature when producing a foam, which will be described below, also is in a suitable range.

When an adjuvant in addition to the compound represented by general formula (1) is contained in the gas generating agent of the present embodiment, the content of the compound represented by general formula (1) is preferably 0.5 to 95 mass% based on the total mass of the compound represented by general formula (1) and the adjuvant.

The oxidizers are not particularly limited, and an example is sodium percarbonate. One oxidizer may be used singly, or two or more may be used in combination.

In the gas generating agent of the present embodiment, the content of the oxidizer is preferably 0.5 to 95 mass% and more preferably 5 to 50 mass% based on the total mass of the compound represented by general formula (1) and the adjuvant.

The crosslinking agents (C) are not particularly limited, and examples include dicumyl peroxide, di-t-butyl peroxide, 2,5-dimethyl-2,5-di-(t-butylperoxy)hexane, 2,5-dimethyl-2,5-di-(t-butylperoxy)hexyne, 1,3-bis(t-butylperoxyisopropyl)benzene, 1,1-bis(t-butylperoxy)-3,3,5-trimethylcyclohexane, 1,1-bis[t-butylperoxy]cyclohexane, n-butyl-4,4-bis(t-butylperoxy)valerate, benzoyl peroxide, p-chlorobenzoyl peroxide, 2,4-dichlorobenzoyl peroxide, t-butyl peroxybenzoate, t-butyl perbenzoate, t-butylperoxy isopropyl carbonate, diacetyl peroxide, lauroyl peroxide, and t-butyl cumyl peroxide. One crosslinking agent (C) may be used singly, or two or more may be used in combination.

In the gas generating agent of the present embodiment, the content of the crosslinking agent (C) is preferably 0.1 to 10 mass% and more preferably 0.5 to 1.5 mass% based on the total mass of the compound represented by general formula (1) and the adjuvant.

The nitrous acid salts are not particularly limited, and examples include sodium nitrite, potassium nitrite, and calcium nitrite. One selected from these can be used singly, or two or more can be used in combination. These nitrous acid salts preferably are pulverized fine powder.

In the gas generating agent of the present embodiment, the content of a nitrous acid salt is preferably 0.5 to 60 mass% and more preferably 15 to 55 mass% based on the total mass of the compound represented by general formula (1) and the adjuvant.

The hydrotalcite is a crystalline composite metal hydroxide, and hydrotalcite represented by general formula (H) below is preferable.

[m²⁺₁₋ₓM³⁺ₓ(OH)₂]^{x+} [Aⁿ⁻_{x/n}·mH₂O]^{x-} (H)

In general formula (H) above, M²⁺ is a divalent metal ion of a metal selected from the group consisting of Mg, Mn, Fe, and Zn, M³⁺ is a trivalent metal ion of a metal selected from the group consisting of Al, Fe, and Cr, Aⁿ⁻ is an anion having a valency of n selected from the group consisting of OH, F, Cl, Br, NO₃, CO₃, and SO₄, x is in a range of 0<x≤0.33, n is an integer, and m is 0 or greater.

Here, m is preferably 0 but varies depending of the dryness and the state of storage of hydrotalcite, and thus m is not particularly limited as long as the effect of the present invention is not impaired. And, n is the valency of an anion and is preferably 1 or 2 and more preferably 2.

Among these, hydrotalcite in which M²⁺ is Mg²⁺ and M³⁺ is Al³⁺ is preferable, and the molar ratio of Al:Mg is preferably 2:5 to 2:10 in terms of availability. For example, when the molar ratio of Al:Mg is 2:5, the molar fraction x of Al (x=Al/(Mg+Al)) is 0.29, and when the molar ratio of Al to Mg is 2:10, the molar fraction x of Al is 0.17.

The hydrotalcite acts to enhance the reactivity of a nitrous acid salt. Blending hydrotalcite makes it possible to promote the degradation of ammonia gas that may be generated during the foaming of the gas generating agent, enhance the reactivity of a nitrous acid salt to thereby suppress the generation of nitrous acid gas, and promote the generation of nitrogen gas to enhance the foamability of the gas generating agent. Although the particle diameter of the hydrotalcite is not particularly limited, increased dispersibility in the gas generating agent is preferable for causing hydrotalcite to effectively act on the reaction between the compound represented by general formula (1) and a nitrous acid salt, and fine-powder hydrotalcite having a maximum particle diameter of 80 µm or less is more preferable.

In the gas generating agent of the present embodiment, the content of the hydrotalcite is preferably 1 to 40 mass% and more preferably 5 to 25 mass% based on the total mass of the compound represented by general formula (1) and the adjuvant.

Various stabilizers, pigments/fillers, foaming regulators, may be further blended with the gas generating agent of the present embodiment as long as the effect of the present invention is not impaired. Stabilizers are not particularly limited, and examples include tribasic lead sulphate, dibasic phosphorous acid salts, lead stearate, zinc stearate, zinc carbonate, zinc oxide, aluminum stearate, dibutyltin malate, and urea. Examples of pigments/fillers are not particularly limited, and examples include chrome yellow, carbon black, titanium dioxide, and calcium carbonate. Foaming regulators are not particularly limited, and examples include maleic acid.

A method for producing the gas generating agent of the present embodiment is not particularly limited, and a commonly used mixing method can be used. For example, the compound represented by general formula (1), a nitrous acid salt, and hydrotalcite may be mixed to be uniformly dispersed under conditions including a temperature of 60°C or less and a time of about 5 minutes using a high speed mixer, a ribbon blender, a cone blender.

The gas generating agent of the present embodiment may be a microcapsule-based gas generating agent.

In the present embodiment, a microcapsule-based gas generating agent refers to a gas generating agent having a core-shell structure.

A specific example of the microcapsule-based gas generating agent is a microcapsule-based gas generating agent having as a core component the compound represented by general formula (1) or a composition containing the compound represented by general formula (1) and further the adjuvant. The main component of the shell of the microcapsule-based gas generating agent is not particularly limited, and an example is polymethyl methacrylate, and a fatty acid salt or a surfactant as a dispersant which will be described below is preferably contained. Introducing air bubbles into a rubber or a thermoplastic resin using such a microcapsule-based gas generating agent makes it possible to introduce uniform air bubbles into a polymer without inhibiting vulcanization and crosslinking and, moreover, without generating degradation residues and VOC components, thereby a decrease in crosslinking density is suppressed, and a crosslinked foam having a good air bubble state and excellent foam characteristics can be obtained.

Although the polymethyl methacrylate is preferably a homopolymer having a methyl methacrylate monomer as a constitutional unit, it may be copolymerized with another monomer as long as the effect of the present invention is not impaired. Another monomer is not particularly limited, and examples include acrylic acid esters such as methyl acrylate, ethyl acrylate, butyl acrylate, and dicyclopentenyl acrylate, methacrylic acid esters such as ethyl methacrylate, butyl methacrylate, and isobornyl methacrylate, acrylonitrile, methacrylonitrile, vinylidene chloride, vinyl chloride, styrene, vinyl acetate, α-methylstyrene, chloroprene, neoprene, and butadiene.

The crosslinking agent (D) used when producing the microcapsule-based gas generating agent is not particularly limited, and examples include (poly)ethylene glycol, divinylbenzene, ethylene glycol di(meth)acrylate, triethylene glycol di(meth)acrylate, allyl methacrylate, triallyl isocyanate, triacrylformal, trimethylolpropane tri(meth)acrylate, 1,3-butylglycol dimethacrylate, and pentaerythritol tri(meth)acrylate. In particular, ethylene glycol dimethacrylate, (poly)ethylene glycol, and trimethylolpropane tri(meth)acrylate are preferable.

A preferable range of the proportion of the crosslinking agent (D) added is 1 to 5 mass% based on the mass of the microcapsule shell monomers (the total mass of the methyl methacrylate monomer and other monomers). When the proportion of the crosslinking agent (D) is less than 1 mass%, the crosslinking of the capsule shell is insufficient, degradation residues generated when the core component degrades cannot be efficiently adsorbed, and therefore degradation residues readily pass through the capsule shell. When the proportion of the crosslinking agent (D) exceeds 5 mass%, the capsule shell becomes hard and brittle, the capsule shell breaks during the degradation of the core component, and the effect of adsorbing degradation residues is decreased.

A polymerization initiator used when producing the microcapsule-based gas generating agent is not particularly limited, and those that are commonly used in this field can be used. Examples of polymerization initiators that can be used include dialkyl peroxides, diacyl peroxides, peroxy esters, peroxydicarbonates, and azo compounds. Specific examples include dialkyl peroxides such as methylethyl peroxide, di-t-butyl peroxide, and dicumyl peroxide, diacyl peroxides such as isobutyl peroxide, benzoyl peroxide, 2,4-dichlorobenzoyl peroxide, and 3,5,5-trimethylhexanoyl peroxide, peroxy esters such as t-butyl peroxypivalate, t-hexyl peroxypivalate, t-butyl peroxyneodecanoate, t-hexyl peroxyneodecanoate, 1-cyclohexyl-1-methylethyl peroxyneodecanoate, 1,1,3,3-tetramethylbutyl peroxyneodecanoate, cumyl peroxyneodecanoate, and (α,α-bis-neodecanoylperoxy)diisopropylbenzene, peroxydicarbonates such as bis(4-t-butylcyclohexyl) peroxydicarbonate, di-n-propyl-peroxydicarbonate, diisopropyl peroxydicarbonate, and di-(3-methyl-3-methoxybutyl) peroxydicarbonate, persulfuric acid salts such as potassium persulfate and ammonium persulfate, and azo compounds such as 2,2'-azobisisobutyronitrile, 2,2'-azobis(2,4-dimethylvaleronitrile), 2,2'-azobis(4-methoxy-2,4-dimethylvaleronitrile), and 1,1'-azobis(1-cyclohexanecarbonitrile).

The microcapsule-based gas generating agent can be produced by an emulsion polymerization method such as a suspension polymerization method. For example, a solid-phase (S phase) core component (for example, the compound represented by general formula (1) or a composition containing the compound represented by general formula (1) and further the adjuvant) is added to an oil phase (O phase) having a monomer (for example, a methyl methacrylate monomer) for a microcapsule shell, a crosslinking agent (D), and a polymerization initiator, and stirring/mixing is performed to thereby prepare a S/O emulsion. This S/O emulsion is added to a water phase (W phase) and stirred/mixed to thereby prepare a S/O/W suspension. This S/O/W suspension is introduced into a pressure polymerizer such as an autoclave and subjected to pressure polymerization to obtain a cake-like substance. This cake-like substance is filtered off by a commonly used method such as filtration or centrifugation, the resulting residue is washed with distilled water about 3 to 5 times, and then dried at a temperature of 40 to 60°C all day and night using a dryer, and it is thus possible to obtain the microcapsule-based gas generating agent. As for pressure polymerization conditions, commonly used conditions can be used, and, for example, the polymerization temperature is 40 to 70°C, the polymerization time is 10 to 24 hours, and the polymerization pressure is 0.2 to 0.3 MPa.

Suspension polymerization is preferably performed in the presence of a dispersion stabilizer and a dispersion stability aid. The dispersion stabilizer is not particularly limited, and, for example, silica, calcium phosphate, magnesium hydroxide, aluminum hydroxide, ferric hydroxide, barium sulfate, calcium sulfate, sodium sulfate, sodium chloride, calcium oxalate, calcium carbonate, barium carbonate, and magnesium carbonate can be used. In particular, silica and calcium phosphate are preferable.

The dispersion stability aid is not particularly limited, and, for example, a condensate of diethanolamine and aliphatic dicarboxylic acid, a condensate of urea and formaldehyde, polyvinylpyrrolidone, polyethylene oxide, polyethyleneimine, tetramethylammonium hydroxide, gelatin, methylcellulose, polyvinyl alcohol, dioctyl sulfosuccinate, polyglycerin fatty acid ester, and sorbitan ester can be used.

A preferable combination of a dispersion stabilizer and a dispersion stability aid is a combination of colloidal silica and a condensate. The condensate is preferably a condensate of diethanolamine and aliphatic dicarboxylic acid, and, in particular, a condensate of diethanolamine and adipic acid and a condensate of diethanolamine and itaconic acid are preferable. A condensate is specified by the acid value, and an acid value of 65 or more and 90 or less is preferable. Moreover, the addition of an inorganic salt, in particular, sodium chloride, sodium sulfate, is suitable for obtaining a uniform microcapsule-based gas generating agent having a small particle diameter.

The microcapsule-based gas generating agent preferably contains a fatty acid salt or a surfactant as a dispersant. This fatty acid salt or surfactant is added to the oil phase (O phase) having a monomer for the microcapsule shell, a crosslinking agent (D), and a polymerization initiator when producing the microcapsule-based gas generating agent. The content of the fatty acid salt or surfactant in the microcapsule-based gas generating agent is preferably 0.01 to 3 mass% based on the core component (the compound represented by general formula (1) or a composition containing the compound represented by general formula (1) and further the adjuvant). When the content is 0.01 mass% or more, there is a tendency that the core component is sufficiently stable, thus the coagulation of solid phase/oil phase droplets is suppressed, and aggregates are unlikely to be formed during polymerization. When the content is 3 mass% or less, there is a tendency that the viscosity of solid phase/oil phase droplets does not become excessively high, droplets are unlikely to adhere to each other, and a broadening of the particle size distribution of microcapsule particles is suppressed.

The fatty acid salt is not particularly limited, and examples include fatty acid amides such as stearic acid amide and arachidic acid amide, zinc stearate, calcium stearate, potassium stearate, aluminum stearate, lithium stearate, sodium stearate, magnesium stearate, zinc palmitate, zinc myristate, calcium palmitate, and sodium palmitate. Among these fatty acid salts, one or more selected from higher fatty acid salts having 15 to 22 carbon atoms are preferable, and one or more higher fatty acid salts of metals selected from the group consisting of potassium, calcium, lithium, and magnesium are more preferable. Titanium oxide, zinc oxide, talc, calcium carbonate, may be used as long as the effect of the present invention is not impaired.

The surfactant is not particularly limited, and includes known cationic surfactants, anionic surfactants, ampholytic surfactants, and nonionic surfactants. Cationic surfactants are not particularly limited, and examples include alkyltrimethylammonium salts, dialkyldimethylammonium salts, and alkyldimethylbenzylammonium salts. Anionic surfactants are not particularly limited, and examples include sulfonate-type anionic surfactants such as alkylbenzenesulfonates, alkylsulfosuccinates, and allylsulfonates, sulfate-type anionic surfactants such as alkylsulfates and polyoxyethylenealkylsulfates, and lignosulfites.

Nonionic surfactants are not particularly limited, and examples include sugar ester-type nonionic surfactants such as sorbitan fatty acid esters and polyoxyethylene sorbitan fatty acid esters, fatty acid ester-type nonionic surfactants such as polyoxyethylene fatty acid esters, vegetable oil-type nonionic surfactants such as polyoxyethylene castor oil, alcohol-type nonionic surfactants such as polyoxyethylene alkyl ethers, alkylphenol-type nonionic surfactants such as polyoxyethylene alkyl (C8-12) phenyl ether-formalin condensates, polyoxyethylene-polyoxypropylene block polymer-type nonionic surfactants such as polyoxyethylene-polyoxypropylene block polymers, and polyaromatic-type nonionic surfactants such as phenyl phenyl ether.

Among the surfactants, nonionic surfactants are most suitable. It is also possible to use anionic surfactants, cationic surfactants, and various additives in combination as long as the effect of the present invention is not impaired.

A preferable range of the HLB value (Hydrophile-Lipophile Balance) of the surfactants is 0.01 to 16, more preferably 0.01 to 9, and even more preferably 0.01 to 3. When the HLB value exceeds 16, the surfactants have strong hydrophilicity, and the solid phase in solid phase/oil phase droplets has an affinity for the aqueous phase. When the solid phase of solid phase/oil phase droplets has an affinity for the aqueous phase, the core component is concentrated near the surface of polymerized microcapsule particles, and the effect of reducing degradation residues is small when the core component degrades. When the HLB value is 16 or less, the solid phase in solid phase/oil phase droplets is uniformly dispersed in the oil phase, and thus the effect of reducing degradation residues is large. In particular, when the HLB value is 3 or less, the surfactants are highly lipophilic, the solid phase in solid phase/oil phase droplets is concentrated in the center of the oil phase, microcapsules in which the core component is concentrated in the center of particles after polymerization are obtained, and also the effect of reducing degradation residues during heating is large.

The foamable composition of the present embodiment comprises the above-described gas generating agent and a material to be foamed.

In the foamable composition of the present embodiment, the material to be foamed that is contained together with the above-described gas generating agent is not particularly limited, and examples include thermoplastic resins and/or rubbers. One material to be foamed may be used singly, or two or more may be used in combination. Examples of the thermoplastic resins include, but are not limited to, vinyl chloride resins, vinyl chloride copolymer resins, polyethylene, polypropylene, and polyolefin copolymer resins represented by ethylene-propylene copolymers, polystyrene resins, and acrylonitrile-butadiene-styrene copolymers (ABS resins).

The foamable composition of the present embodiment may further comprise a crosslinking agent (E). The crosslinking agent (E) contained in the foamable composition of the present embodiment may be the same as or different from the crosslinking agent (C) contained in the above-described gas generating agent.

The foam of the present embodiment is a foam obtained by foaming the above-described foamable composition.

A method for producing the foam of the present embodiment is not particularly limited as long as the method comprises a step of heating a foamable composition comprising the above-described gas generating agent and a material to be foamed, and a commonly used method for producing a foam can be used. For example, a foamable composition (an unfoamed resin composition) can be prepared by kneading a thermoplastic resin, a crosslinking agent (E), and the above-described gas generating agent with heated rolls. The kneading temperature is preferably 90 to 130°C. Here, the crosslinking agent (E) contained in the foamable composition is not particularly limited, and examples include dicumyl peroxide, di-t-butyl peroxide, 2,5-dimethyl-2,5-di-(t-butylperoxy)hexane, 2,5-dimethyl-2,5-di-(t-butylperoxy)hexyne, 1,3-bis(t-butylperoxyisopropyl)benzene, 1,1-bis(t-butylperoxy)-3,3,5-trimethylcyclohexane, 1,1-bis[t-butylperoxy]cyclohexane, n-butyl-4,4-bis(t-butylperoxy)valerate, benzoyl peroxide, p-chlorobenzoyl peroxide, 2,4-dichlorobenzoyl peroxide, t-butyl peroxybenzoate, t-butyl perbenzoate, t-butylperoxy isopropyl carbonate, diacetyl peroxide, lauroyl peroxide, and t-butyl cumyl peroxide. One crosslinking agent (E) may be used singly, or two or more may be used in combination. The content of crosslinking agent (E) is preferably 0.1 to 10 parts by mass and more preferably 0.5 to 1.5 parts by mass based on 100 parts by mass of the thermoplastic resin.

The unfoamed resin composition thus obtained is introduced into a metal mold, pressurized with a press, and thereby a foam of a resin composition (for example, a thermoplastic resin) is obtained. The metal mold thickness, pressurizing conditions, are not particularly limited, and a conventionally known foam molding method can be suitably employed according to the kind, the application, of the thermoplastic resin. For example, the unfoamed resin composition is introduced into a metal mold having a thickness of 1 to 30 mm to an extent of 100%, pressurized with a press for 3 to 60 minutes under 100 to 170°C and 150 kg/cm² conditions, and water-cooled for 3 to 60 minutes, and thereby a sheet-like composition is obtained. The resulting sheet-like composition is left to stand still for one day, then heated for 1 hour in an oven at 40 to 80°C, and heated at 130 to 250°C for 150 to 600 seconds, and a foam of the resin composition is obtained.

When producing a foam of the resin composition in this way, the amount of the above-described gas generating agent used can be suitably selected according to the intended expansion ratio and is not particularly limited, but preferably 1 to 30 parts by mass of the gas generating agent is blended based on 100 parts by mass of a thermoplastic resin.

Examples of rubbers used in the present embodiment include, but are not limited to, natural rubber (NR), polyisoprene rubber, styrene-butadiene rubber (SBR), acrylonitrile-butadiene rubber, chloroprene rubber, ethylene-propylene rubber, ethylene-propylene-diene rubber, and butadiene rubber.

As a method in which the above-described gas generating agent is blended with a rubber to prepare a foamable composition and a foam is produced therefrom, commonly used foam production conditions can be used. For example, a rubber material, a vulcanizing agent, a filler, a vulcanization accelerator, and the above-described gas generating agent are uniformly dispersed with kneading rolls to obtain a foamable composition. The resulting foamable composition is fed to an extruder heated to 70 to 90°C to prepare an unvulcanized molding. The resulting unvulcanized molding is heated for 5 to 15 minutes in an oven heated to 60 to 220°C to perform vulcanization and foaming, and a foam of the rubber material is obtained.

Although the proportion of each component blended in the foamable composition is not particularly limited, preferably 0.1 to 10 parts by mass of the vulcanizing agent is blended based on 100 parts by mass of the rubber material. Preferably 10 to 150 parts by mass of the filler is blended based on 100 parts by mass of the rubber material. Preferably 0.1 to 20 parts by mass of the vulcanization accelerator is blended based on 100 parts by mass of the rubber material.

When producing a foam of the rubber material in this way, the amount of the above-described gas generating agent used can be suitably selected according to the intended expansion ratio and is not particularly limited, but preferably 1 to 20 parts by mass of the gas generating agent is blended based on 100 parts by mass of the rubber.

Specific examples of the vulcanizing agent used in the present embodiment are not particularly limited, and an example is sulfur.

Specific examples of the filler used in the present embodiment are not particularly limited, and examples include heavy and light calcium carbonates, and carbon black.

Specific examples of the vulcanization accelerator used in the present embodiment are not particularly limited, and examples include dithiocarbamic acid salt-based vulcanization accelerators and dithiocarbamic acid-based vulcanization accelerators, and specifically examples include DM (dibenzylthiazole disulfide), zinc dimethyldithiocarbamate, and zinc dibutyldithiocarbamate.

The foamable composition of the present embodiment may contain other additives. Other additives are not particularly limited, and examples include Diana Process Oil, zinc oxide, stearic acid, calcium oxide, urea-based auxiliaries, silicon dioxide (silica), talc, magnesium oxide, zinc stearate, calcium hydroxide, barium stearate, dibasic lead phosphite, and lead oxide.

### Examples

The present invention is described in more detail by way of Examples and Comparative Examples below, but the present invention is not at all limited to the Examples. In Examples and Comparative Examples provided below, unless specified otherwise, the structure of a compound used for a gas generating agent was identified by ¹H-NMR and the melting point, and the degradation starting temperature of a gas generating agent, the amount of generated gas, and the amount of generated ammonia were respectively evaluated by the following methods.

### <Measurement of degradation starting temperature of gas generating agent>

The degradation starting temperature of a gas generating agent was measured using a differential thermal-thermogravimetric simultaneous analyzer (EXSTAR 6000 manufactured by SII NanoTechnology Inc.) in air under conditions where the starting temperature was 25°C and the temperature was increased at 10°C/min. The temperature at which the weight was lower by 5% than the weight at the beginning of increasing the temperature was regarded as the degradation starting temperature.

### <Measurement of amount of generated gas>

After 0.5 g of a gas generating agent was placed in a test tube and 10 mL of liquid paraffin as a heating medium was added, the test tube and a gas buret were connected by a rubber tube, and the test tube was immersed in an oil bath at 60°C. Then, the oil bath was heated to 240°C at a heating rate of 2°C/min. Gas generated during this heating was all captured by the gas buret to determine the amount (mL) of generated gas per gram of the gas generating agent.

### <Measurement of amount of generated ammonia>

After 0.5 g of a gas generating agent was placed in a test tube and 10 mL of liquid paraffin was added as a heating medium, this test tube, a screw-cap test tube, and a screw-cap bottle having 100 mL of 0.1 N hydrochloric acid were connected by a rubber tube in this order. While allowing nitrogen gas to flow at a flow rate of 0.4 L/min, the test tube containing the gas generating agent was heated to 228°C in a block heater. Ammonia gas generated during this heating was captured by 0.1 N hydrochloric acid, and ammonium ions in hydrochloric acid were quantified by an ion chromatograph (DX-320J manufactured by Nippon Dionex K.K.) to determine the amount (mg) of generated ammonia gas per gram of the gas generating agent.

### (Synthesis Example 1) Synthesis of semicarbazonoacetic acid (5)

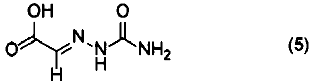

To a 30 mL recovery flask were added 1.33 g (12 mmol) of semicarbazide hydrochloride (manufactured by Kanto Chemical Co., Inc.) and 6 mL of water, and the mixture was stirred at room temperature using a magnetic stirrer until a homogeneous solution was formed, and then 1.91 g (13 mmol) of a 50 mass% aqueous glyoxylic acid solution (manufactured by Tokyo Chemical Industry Co., Ltd.) was added. White solids precipitated shortly after adding glyoxylic acid, and after stirring for 1.0 hour, the reaction solution was filtered, and water-washing and then vacuum-drying at 50°C for 17 hours were performed, thus giving 1.458 g (11 mmol) of white solids. The resulting solids were analyzed by ¹H-NMR and confirmed as semicarbazonoacetic acid (5) (¹H-NMR (DMSO-d₆, 500 MHz, δ; ppm) = 6.6-6.9 (br; 2H), 7.1 (s; 1H), 10.9 (s; 1H)). The molar yield was 92%. The melting point of the resulting solids measured using a micro melting point apparatus BY-1 (manufactured by Yazawa Scientific Co., Ltd.) was 223°C.

### (Synthesis Example 2) Synthesis of carbohydrazidediacetic acid (8)

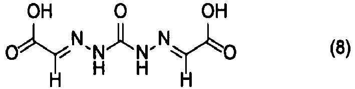

To a 30 mL recovery flask were added 1.07 g (12 mmol) of carbohydrazide (manufactured by Wako Pure Chemical Industries, Ltd.) and 12 mL of water, and the mixture was stirred at room temperature using a magnetic stirrer until a homogeneous solution was formed, and then 3.840 g (26 mmol) of a 50 mass% aqueous glyoxylic acid solution (manufactured by Tokyo Chemical Industry Co., Ltd.) was added. White solids precipitated shortly after adding glyoxylic acid, and after stirring for 1.0 hour, the reaction solution was filtered, and water-washing and then vacuum-drying at 50°C for 17 hours were performed, thus giving 2.52 g (11 mmol) of white solids. The resulting solids were analyzed by ¹H-NMR and confirmed as carbohydrazidediacetic acid (8) (¹H-NMR (DMSO-d₆, 500 MHz, δ; ppm) = 7.4 (s; 2H), 11.5 (s; 2H)). The molar yield was 93%. The melting point of the resulting solids measured using a micro melting point apparatus BY-1 (manufactured by Yazawa Scientific Co., Ltd.) was 207 to 208°C.

### (Synthesis Example 3) Synthesis of (methoxycarbonyl)hydrazonoacetic acid (7-1)

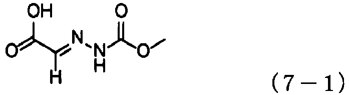

To a 30 mL recovery flask were added 8.41 g (93 mmol) of methyl carbazate (manufactured by Tokyo Chemical Industry Co., Ltd.) and 10 mL of 0.1 N hydrochloric acid and the mixture was stirred at room temperature using a magnetic stirrer until a homogeneous solution was formed, and then 14.38 g (97 mmol) of a 50 mass% aqueous glyoxylic acid solution (manufactured by Tokyo Chemical Industry Co., Ltd.) was added. White solids gradually precipitated after adding glyoxylic acid, and after stirring for 24 hours, the reaction solution was filtered, and washing with 10 mL of water and then vacuum-drying at 50°C for 18 hours were performed, thus giving 11.043 g (76 mmol) of white solids. The resulting solids were analyzed by ¹H-NMR and confirmed as (methoxycarbonyl)hydrazonoacetic acid (7-1) (¹H-NMR (DMSO-d₆, 500 MHz, δ; ppm) = 3.7 (s; 3H), 7.3 (s; 1H), 11.5-11.7 (br)). The molar yield was 82%. The melting point of the resulting solids measured using a micro melting point apparatus BY-1 (manufactured by Yazawa Scientific Co., Ltd.) was 181°C.

### (Synthesis Example 4) Synthesis of (ethoxycarbonyl)hydrazonoacetic acid (7-2)

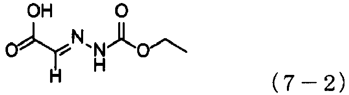

To a 30 mL recovery flask were added 1.04 g (10 mmol) of ethyl carbazate (manufactured by Tokyo Chemical Industry Co., Ltd.) and 1 mL of water, 0.1 mL of concentrated hydrochloric acid was added, the mixture was stirred at room temperature using a magnetic stirrer, and then 1.69 g (11 mmol) of a 50 mass% aqueous glyoxylic acid solution (manufactured by Tokyo Chemical Industry Co., Ltd.) was added. White solids precipitated shortly after adding glyoxylic acid, and after stirring for 18 hours, the reaction solution was filtered, and water-washing and then vacuum-drying at 50°C for 21 hours were performed, thus giving 1.02 g (6.4 mmol) of white solids. The resulting solids were analyzed by ¹H-NMR and confirmed as (ethoxycarbonyl)hydrazonoacetic acid (7-2) (¹H-NMR (DMSO-d₆, 500 MHz, δ; ppm) = 1.2 (t; 3H), 4.1 (q; 2H), 7.3 (s; 1H), 11.5-11.7 (br)). The molar yield was 64%. The melting point of the resulting solids measured using a micro melting point apparatus BY-1 (manufactured by Yazawa Scientific Co., Ltd.) was 173 to 174°C.

### (Synthesis Example 5) Synthesis of (t-butoxycarbonyl)hydrazonoacetic acid (7-3)

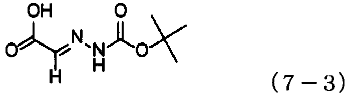

To a 30 mL recovery flask were added 1.04 g (7.9 mmol) of t-butyl carbazate (manufactured by Tokyo Chemical Industry Co., Ltd.) and 3 mL of water and the mixture was stirred at room temperature using a magnetic stirrer, and then 1.20 g (8.1 mmol) of a 50 mass% aqueous glyoxylic acid solution (manufactured by Tokyo Chemical Industry Co., Ltd.) was added. White solids precipitated shortly after adding glyoxylic acid, and after stirring for 1.0 hour, the reaction solution was filtered, and water-washing and then vacuum-drying at 50°C for 17 hours were performed, thus giving 1.07 g (5.7 mmol) of white solids. The resulting solids were analyzed by ¹H-NMR and confirmed as (t-butoxycarbonyl)hydrazonoacetic acid (7-3) (¹H-NMR (DMSO-d₆, 500 MHz, δ; ppm) = 1.5 (s; 9H), 7.3 (s; 1H), 11.2-11.3 (br)). The molar yield was 72%. The melting point of the resulting solids measured using a micro melting point apparatus BY-1 (manufactured by Yazawa Scientific Co., Ltd.) was 139 to 140°C.

### (Synthesis Example 6) Synthesis of (amidino)hydrazonoacetic acid (6)

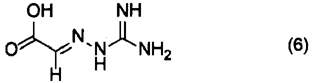

To a 30 mL recovery flask were added 1.322 g (12 mmol) of aminoguanidine hydrochloride (manufactured by Tokyo Chemical Industry Co., Ltd.) and 6 mL of 0.1 N hydrochloric acid and the mixture was stirred at room temperature using a magnetic stirrer until a homogeneous solution was formed at room temperature, and then 1.777 g (12 mmol) of a 50 mass% aqueous glyoxylic acid solution was added. White solids precipitated shortly after adding glyoxylic acid, and after stirring for 2.5 hour, the reaction solution was filtered, and water-washing and then vacuum-drying at 50°C for 4 hours were performed, thus giving 1.545 g (10 mmol) of white solids. The resulting solids were analyzed by ¹H-NMR and confirmed as (amidino)hydrazonoacetic acid (6), which is a condensate of aminoguanidine and glyoxylic acid (¹H-NMR (DMSO-d₆, 500 MHz, δ; ppm) = 7.1 (s; 1H)). The molar yield was 83%. The resulting solids degraded at 145°C when the melting point was measured using a micro melting point apparatus BY-1 (manufactured by Yazawa Scientific Co., Ltd.).

### (Comparative Synthesis Example 1) Synthesis of glyoxal bis[(methoxycarbonyl)hydrazone] (9)

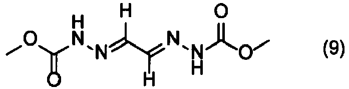

To a 100 mL recovery flask were added 1.064 g (12 mmol) of methyl carbazate (manufactured by Tokyo Chemical Industry Co., Ltd.) and 3 mL of 0.1 N hydrochloric acid and stirred for 5 minutes to completely dissolve methyl carbazate. Then, 877 mg (5.9 mmol) of a 39% aqueous glyoxal solution (manufactured by Tokyo Chemical Industry Co., Ltd.) was added, and the mixture was stirred at room temperature using a magnetic stirrer. White precipitates were generated when stirring was started, and after stirring for 2 hours, the reaction solution was filtered, and washing with water and methanol and then vacuum-drying at 50°C for 18 hours were performed, thus giving 936 mg (4.6 mmol) of white solids. The resulting solids were analyzed by ¹H-NMR and confirmed as glyoxal bis[(methoxycarbonyl)hydrazone] (9) (¹H-NMR (DMSO-d₆, 500 MHz, δ; ppm) = 3.7 (s, 6H), 7.7 (s, 2H), 11.4-11.5 (br)). The molar yield was 79%. The resulting solids degraded at 268 to 270°C when the melting point was measured using a micro melting point apparatus BY-1 (manufactured by Yazawa Scientific Co., Ltd.).

### (Comparative Synthesis Example 2) Synthesis of methyl 1-methylidenecarbazate (10)

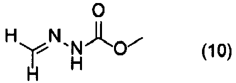

To a 100 mL recovery flask were added 1.361 g (15 mmol) of methyl carbazate (manufactured by Tokyo Chemical Industry Co., Ltd.) and 1.30 g (16 mmol) of a 37% aqueous formaldehyde solution (manufactured by Tokyo Chemical Industry Co., Ltd.) and the mixture was stirred at room temperature using a magnetic stirrer. White precipitates were generated when stirring was started, and after stirring for 3 hours, the reaction solution was filtered, and water-washing and then vacuum-drying at 50°C for 16 hours were performed, thus giving 1.243 g (12 mmol) of white solids. The resulting solids were analyzed by ¹H-NMR and confirmed as methyl 1-methylidenecarbazate (¹H-NMR (DMSO-d₆, 500 MHz, δ; ppm) = 3.6 (s, 3H), 3.8 (s, 2H), 8.7-8.9 (br)). The molar yield was 82%. The resulting solids degraded at 129 to 131°C when the melting point was measured using a micro melting point apparatus BY-1 (manufactured by Yazawa Scientific Co., Ltd.).

### (Example 1)

The degradation starting temperature of semicarbazonoacetic acid (5) obtained in Synthesis Example 1 and the amount of generated gas were measured. The degradation starting temperature and the amount of generated gas are shown in Table 1.

### (Example 2)

The degradation starting temperature of carbohydrazidediacetic acid (8) obtained in Synthesis Example 2 and the amount of generated gas were measured under the same conditions as in Example 1. The degradation starting temperature and the amount of generated gas are shown in Table 1.

### (Example 3)

The degradation starting temperature of (methoxycarbonyl)hydrazonoacetic acid (7-1) obtained in Synthesis Example 3 and the amount of generated gas were measured under the same conditions as in Example 1. The degradation starting temperature and the amount of generated gas are shown in Table 1.

### (Example 4)

The degradation starting temperature of (ethoxycarbonyl)hydrazonoacetic acid (7-2) obtained in Synthesis Example 4 and the amount of generated gas were measured under the same conditions as in Example 1. The degradation starting temperature and the amount of generated gas are shown in Table 1.

### (Example 5)

The degradation starting temperature of (t-butoxycarbonyl)hydrazonoacetic acid (7-3) obtained in Synthesis Example 5 and the amount of generated gas were measured under the same conditions as in Example 1. The degradation starting temperature and the amount of generated gas are shown in Table 1.

### (Example 6)

The degradation starting temperature of (amidino)hydrazonoacetic acid (6) obtained in Synthesis Example 6 and the amount of generated gas were measured under the same conditions as in Example 1. The degradation starting temperature and the amount of generated gas are shown in Table 1.

### (Comparative Example 1)

The degradation starting temperature of semicarbazide hydrochloride (manufactured by Tokyo Chemical Industry Co., Ltd.) and the amount of generated gas were measured under the same conditions as in Example 1. The degradation starting temperature and the amount of generated gas are shown in Table 1.

### (Comparative Example 2)

The degradation starting temperature of carbohydrazide (manufactured by Wako Pure Chemical Industries, Ltd.) and the amount of generated gas were measured under the same conditions as in Example 1. The degradation starting temperature and the amount of generated gas are shown in Table 1.

### (Comparative Example 3)

The degradation starting temperature of methyl carbazate (manufactured by Tokyo Chemical Industry Co., Ltd.) and the amount of generated gas were measured under the same conditions as in Example 1. The degradation starting temperature and the amount of generated gas are shown in Table 1.

### (Comparative Example 4)

The degradation starting temperature of ethyl carbazate (manufactured by Tokyo Chemical Industry Co., Ltd.) and the amount of generated gas were measured under the same conditions as in Example 1. The degradation starting temperature and the amount of generated gas are shown in Table 1.

### (Comparative Example 5)

The degradation starting temperature of t-butyl carbazate (manufactured by Tokyo Chemical Industry Co., Ltd.) and the amount of generated gas were measured under the same conditions as in Example 1. The degradation starting temperature and the amount of generated gas are shown in Table 1.

### (Comparative Example 6)

The degradation starting temperature of aminoguanidine hydrochloride (manufactured by Tokyo Chemical Industry Co., Ltd.) and the amount of generated gas were measured under the same conditions as in Example 1. The degradation starting temperature and the amount of generated gas are shown in Table 1.

### (Comparative Example 7)

The degradation starting temperature of glyoxal bis[(methoxycarbonyl)hydrazone] (9) obtained in Comparative Synthesis Example 1 and the amount of generated gas were measured under the same conditions as in Example 1. The degradation starting temperature and the amount of generated gas are shown in Table 1.

### (Comparative Example 8)

The degradation starting temperature of methyl 1-methylidenecarbazate (10) obtained in Comparative Synthesis Example 2 and the amount of generated gas were measured under the same conditions as in Example 1. The degradation starting temperature and the amount of generated gas are shown in Table 1.

### (Comparative Example 9)

The degradation starting temperature of 4,4'-oxybis(benzenesulfonyl hydrazide) (OBSH) (manufactured by Eiwa Chemical Ind. Co., Ltd.) and the amount of generated gas were measured under the same conditions as in Example 1. The degradation starting temperature and the amount of generated gas are shown in Table 1.

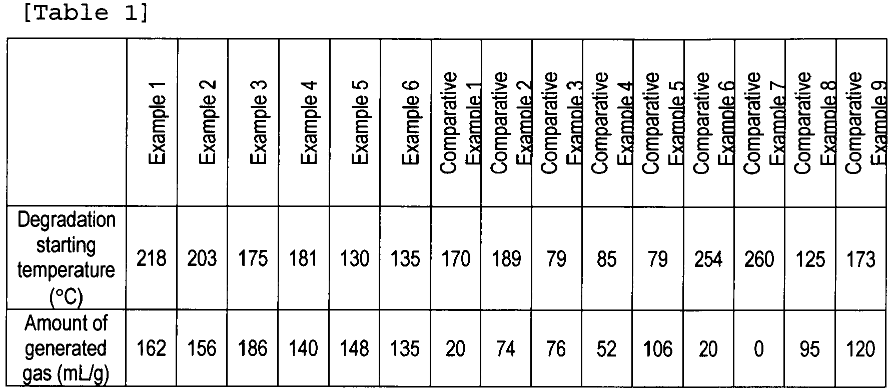

It was found from Table 1 that due to the condensation of hydrazine derivatives such as semicarbazide, carbohydrazide, carbazic acid esters, and aminoguanidine with glyoxylic acid, the amount of generated gas is increased, and also the degradation starting temperature is 130°C or higher, which is a suitable temperature for a chemical blowing agent for general-purpose plastics (for example, thermoplastic resins) and rubbers.

### (Example 7)

The amount of generated ammonia gas of semicarbazonoacetic acid (5) obtained in Synthesis Example 1 was measured by the method described above. The result is shown in Table 2.

### (Example 8)

The amount of generated ammonia gas of carbohydrazidediacetic acid (8) obtained in Synthesis Example 2 was measured under the same conditions as in Example 7 except that the heating temperature of the block heater was changed to 185°C. The result is shown in Table 2.

### (Example 9)

The amount of generated ammonia gas of (methoxycarbonyl)hydrazonoacetic acid (7-1) obtained in Synthesis Example 3 was measured under the same conditions as in Example 7 except that the heating temperature of the block heater was changed to 191°C. The result is shown in Table 2.

### (Example 10)

The amount of generated ammonia gas of (amidino)hydrazonoacetic acid (6) obtained in Synthesis Example 6 was measured under the same conditions as in Example 7 except that the heating temperature of the block heater was changed to 191°C. The result is shown in Table 2.

### (Comparative Example 10)

The amount of generated ammonia gas of azodicarbonamide (ADCA) (manufactured by Eiwa Chemical Ind. Co., Ltd.) was measured under the same conditions as in Example 7 except that the heating temperature of the block heater was changed to 210°C. The result is shown in Table 2.

**[Table 2]**

| | Example 7 | Example 8 | Example 9 | Example 10 | Comparative Example 10 |
|---|---|---|---|---|---|
| Amount of generated ammonia gas (mg/g) | 0.50 | 0.49 | 1.35 | 0.80 | 11.11 |

It was found from Table 2 that the gas generating agents of the present embodiment generate smaller amounts of ammonia gas and, as chemical blowing agents for general-purpose plastics (for example, thermoplastic resins) and rubbers, do not contaminate the metal mold during molding and are highly safe.

### (Example 11)

Polyethylene (trade name "LE200M" manufactured by Japan Polyethylene Corporation) in an amount of 100 parts by mass was kneaded with an open mill heated to 120°C, then 16 parts by mass of semicarbazonoacetic acid (5) obtained in Synthesis Example 1 as a gas generating agent was added and kneaded for 4 minutes and 30 seconds, then 0.8 parts by mass of dicumyl peroxide (DCP) (trade name "Percumyl D" manufactured by NOF Corporation) was added and kneaded for 1 minute and 30 seconds, and the kneaded material was removed from the open mill. The kneaded material was introduced into a metal mold (2 mm) of a press heated to 165°C such that the inner volume was filled 100%, pressurized for 3 minutes at a pressing pressure of 50 kg/cm², then pressurized for 3 minutes at a pressing pressure of 150 kg/cm², and water-cooled for 5 minutes, thus giving a sheet-like composition. The resulting sheet-like composition was left to stand still for one day, heated for 1 hour in an oven at 60°C, and heated at 220°C for 300 seconds, thus giving a foam. The evaluation results of the resulting foam are shown in Table 3.

### (Example 12)

A foam was obtained under the same conditions as in Example 11 except that the gas generating agent was changed to carbohydrazidediacetic acid (8) obtained in Synthesis Example 2, and the time of heating at 220°C was 242 seconds. The evaluation results of the resulting foam are shown in Table 3.

### (Example 13)

A foam was obtained under the same conditions as in Example 11 except that the gas generating agent was changed to (methoxycarbonyl)hydrazonoacetic acid (7-1) obtained in Synthesis Example 3, and the time of heating at 220°C was 300 seconds. The evaluation results of the resulting foam are shown in Table 3.

### (Example 14)

A foam was obtained under the same conditions as in Example 11 except that the gas generating agent was changed to (ethoxycarbonyl)hydrazonoacetic acid (7-2) obtained in Synthesis Example 4, and the time of heating at 220°C was 286 seconds. The evaluation results of the resulting foam are shown in Table 3.

### (Comparative Example 11)

A foam was obtained under the same conditions as in Example 11 except that the gas generating agent was changed to azodicarbonamide (ADCA) (manufactured by Eiwa Chemical Ind. Co., Ltd.), and the time of heating at 220°C was 305 seconds. The evaluation results of the resulting foam are shown in Table 3.

### (Comparative Example 12)

A foam was obtained under the same conditions as in Example 11 except that the gas generating agent was changed to sodium hydrogencarbonate (trade name "Cellborn FE-507", manufactured by Eiwa Chemical Ind. Co., Ltd.) and the time of heating at 220°C was 240 seconds. The evaluation results of the resulting foam are shown in Table 3.

**[Table 3]**

| Example Number | | Example 11 | Example 12 | Example 13 | Example 14 | Comparative Example 11 | Comparative Example 12 |
|---|---|---|---|---|---|---|---|
| Resin | Polyethylene | 100 parts | 100 parts | 100 parts | 100 parts | 100 parts | 100 parts |
| Gas generating agent | ADCA | - | - | - | - | 16 parts | - |
| | Cellborn FE-507 | - | - | - | - | - | 16 parts |
| | Formula (5) | 16 parts | - | - | - | - | - |
| | Formula (8) | - | 16 parts | - | - | - | - |
| | Formula (7-1) | - | - | 16 parts | - | - | - |
| | Formula (7-2) | - | - | - | 16 parts | - | - |
| Crosslinking agent (E) | Percumyl D | 0.8 parts | 0.8 parts | 0.8 parts | 0.8 parts | 0.8 parts | 0.8 parts |
| Foaming time [sec] | | 300 | 242 | 300 | 286 | 305 | 240 |
| Specific gravity [g/cm3] | | 0.081 | 0.105 | 0.120 | 0.118 | 0.032 | 0.318 |
| Expansion ratio | | 11.4 | 8.8 | 7.7 | 7.8 | 28.8 | 2.9 |
| Number of air bubbles n=5 per 1 cm | | 5-9 | 9-13 | 14-23 | 22-29 | 23-30 | 7-11 |
| Color of foam | | Brown | Brown | Brown | Brown | Pale yellow | White |

In the present Examples, the specific gravity, the expansion ratio, and the number of air bubbles of a foam were measured as follows.
Specific gravity: measured with an electronic gravimeter MD-200S.
Expansion ratio: calculated by the specific gravity of polyethylene (0.92 g/cm³)/the specific gravity of a foam.
Number of air bubbles: measured with a microscope HIROX KH7700 2D measurement.

### (Example 15)

Saturated hydrocarbon rubber (trade name "EPT 4021" manufactured by Mitsui Chemicals, Inc.) in an amount of 100 parts by mass was kneaded with a cooled (water-flowed) roll mill, then 70 parts by mass of carbon black (trade name "Asahi #50 UG" manufactured by Asahi Carbon Co., Ltd.), 40 parts by mass of light calcium carbonate (manufactured by Ohmi Chemical Industry Co., Ltd.), 45 parts by mass Diana Process Oil (trade name "PW-90", manufactured by Idemitsu Kosan Co., Ltd.), 5 parts by mass of zinc oxide (ZnO (zinc white) manufactured by Mitsui Mining & Smelting Co., Ltd.), and 1 part by mass of stearic acid (CH₃(CH₂)₁₆COOH, trade name "Tsubaki" manufactured by NOF Corporation) were added and kneaded, thus giving kneaded material A. Kneaded material A was stored for 1 day or more at normal temperature for aging. The aged kneaded material A was kneaded with a cooled (water-flowed) roll mill, then 1.1 parts by mass of a dithiocarbamic acid salt-based vulcanization accelerator (zinc dimethyldithiocarbamate, trade name "Nocceler PZ" manufactured by Ouchi Shinko Chemical Industrial Co., Ltd.), 1.7 parts by mass of a dithiocarbamic acid-based vulcanization accelerator (zinc dibutyldithiocarbamate, trade name "Nocceler BZ" manufactured by Ouchi Shinko Chemical Industrial Co., Ltd.) were added and kneaded, then 1.7 parts by mass of sulfur (trade name "Fine Powder Sulfur S" manufactured by Hosoi Chemical Industry Co., Ltd.), 5 parts by mass of calcium oxide (trade name "F Lime 1300D" manufactured by Calfine Co., Ltd.), 3.84 parts by mass of semicarbazonoacetic acid (5) obtained in Synthesis Example 1 as a gas generating agent, and 6 parts by mass of a urea-based auxiliary (trade name "Cellpaste K5" manufactured by Eiwa Chemical Ind. Co., Ltd.) were added and kneaded, and kneaded material B was removed from the roll mill. Kneaded material B was introduced into an extruder and extruded at a die temperature of 50°C, thus giving a sheet-like molding. The resulting sheet-like molding was aged for 1 day at normal temperature and then heated for 10 minutes in an oven at 180°C, thus giving a form. The expansion ratio of the resulting blowing agent was 1.946. As a blank for calculating the expansion ratio, kneaded material C was produced by the same production method as that for kneaded material B except that no gas generating agent was blended, and the specific gravity of kneaded material C was measured. The expansion ratio was calculated by the specific gravity of kneaded material C (0.860 g/cm³)/the specific gravity of the foam (0.442 g/cm³) . In the present Examples, the specific gravity was measured with an electronic gravimeter MD-200S.

### (Example 16)

A foam was obtained under the same conditions as in Example 15 except that the gas generating agent was changed to 4.84 parts by mass of carbohydrazidediacetic acid (8) obtained in Synthesis Example 2. The expansion ratio of the resulting blowing agent was 1.541. The expansion ratio was calculated by the specific gravity of kneaded material C (0.860 g/cm³)/the specific gravity of the foam (0.558 g/cm³).

### (Example 17)

A foam was obtained under the same conditions as in Example 15 except that the gas generating agent was changed to 3.18 parts by mass of (methoxycarbonyl)hydrazonoacetic acid (7-1) obtained in Synthesis Example 3. The expansion ratio of the resulting blowing agent was 1.274. The expansion ratio was calculated by the specific gravity of kneaded material C (0.860 g/cm³)/the specific gravity of the foam (0.675 g/cm³) .

### (Example 18)

A foam was obtained under the same conditions as in Example 15 except that the gas generating agent was changed to 4.14 parts by mass of (ethoxycarbonyl)hydrazonoacetic acid (7-2) obtained in Synthesis Example 4. The expansion ratio of the resulting blowing agent was 1.509. The expansion ratio was calculated by the specific gravity of kneaded material C (0.860 g/cm³)/the specific gravity of the foam (0.570 g/cm³) .

### (Example 19)

A foam was obtained under the same conditions as in Example 15 except that the gas generating agent was changed to 3.94 parts by mass of (t-butoxycarbonyl)hydrazonoacetic acid (7-3) obtained in Synthesis Example 5. The expansion ratio of the resulting blowing agent was 1.528. The expansion ratio was calculated by the specific gravity of kneaded material C (0.860 g/cm³)/the specific gravity of the foam (0.563 g/cm³) .

## Claims

1. A gas generating agent comprising a compound represented by general formula (1) below: 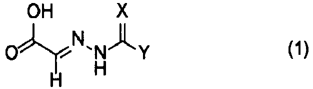 wherein X is an oxygen atom or an imino group, and Y is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (2) below.

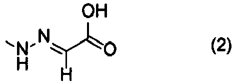

2. The gas generating agent according to claim 1, wherein the compound represented by general formula (1) comprises a structure derived from compound (A) represented by general formula (3) below and a structure derived from glyoxylic acid (B) : 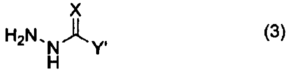 wherein X is an oxygen atom or an imino group, and Y' is an amino group, a hydroxy group, an alkoxy group having 1 to 8 carbon atoms, an allyloxy group having 3 to 8 carbon atoms, a benzyloxy group, an alkenyloxy group having 2 to 8 carbon atoms, an aryloxy group having 6 to 10 carbon atoms, or a group represented by formula (4) below.

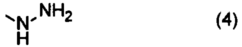

3. The gas generating agent according to claim 1, wherein the compound represented by general formula (1) is a compound represented by formula (5) below.

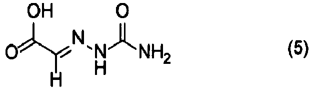

4. The gas generating agent according to claim 1, wherein the compound represented by general formula (1) is a compound represented by formula (6) below.

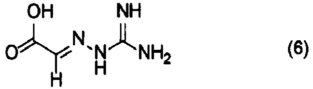

5. The gas generating agent according to claim 1, wherein the compound represented by general formula (1) is a compound represented by general formula (7) below: 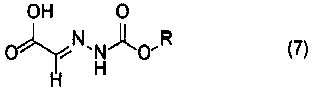 wherein R is a hydrogen atom, an alkyl group having 1 to 8 carbon atoms, an allyl group having 3 to 8 carbon atoms, a benzyl group, an alkenyl group having 2 to 8 carbon atoms, or an aryl group having 6 to 10 carbon atoms.

6. The gas generating agent according to claim 1, wherein the compound represented by general formula (1) is a compound represented by formula (8) below.

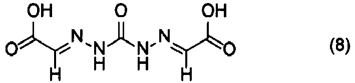

7. The gas generating agent according to any one of claims 1 to 6, wherein the gas generating agent is a microcapsule-based agent.

8. A foamable composition comprising the gas generating agent according to any one of claims 1 to 7 and a material to be foamed.

9. The foamable composition according to claim 8, wherein the material to be foamed is a thermoplastic resin and/or a rubber.

10. A method for producing a foam, comprising a step of heating the foamable composition according to claim 8 or 9.

11. A foam, wherein the foam is obtained by foaming the foamable composition according to claim 8 or 9.

12. A compound represented by formula (8) below.

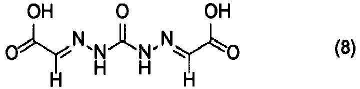

## Patentansprüche

1. Gas-erzeugendes Mittel, umfassend eine Verbindung, dargestellt durch die nachstehende allgemeine Formel (1): 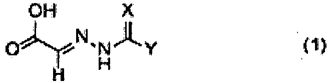 worin X ein Sauerstoffatom oder eine Iminogruppe ist, und Y eine Aminogruppe, eine Hydroxygruppe, eine Alkoxygruppe mit 1 bis 8 Kohlenstoffatomen, eine Allyloxygruppe 3 bis 8 Kohlenstoffatomen, eine Benzyloxygruppe, eine Alkenyloxygruppe mit 2 bis 8 Kohlenstoffatomen, eine Aryloxygruppe mit 6 bis 10 Kohlenstoffatomen oder eine Gruppe, dargestellt durch die nachstehende Formel (2) ist.

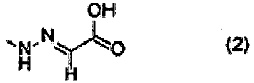

2. Gas-erzeugendes Mittel gemäß Anspruch 1, wobei die Verbindung, dargestellt durch die allgemeine Formel (1), eine Struktur, abgeleitet von Verbindung (A), dargestellt durch die nachstehende allgemeine Formel (3), und eine Struktur, abgeleitet von Glyoxylsäure (B), umfasst: 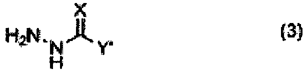 worin X ein Sauerstoffatom oder eine Iminogruppe ist und Y' eine Aminogruppe, eine Hydroxygruppe, eine Alkoxygruppe mit 1 bis 8 Kohlenstoffatomen, eine Allyloxygruppe mit 3 bis 8 Kohlenstoffatomen, eine Benzyloxygruppe, eine Alkenyloxygruppe mit 2 bis 8 Kohlenstoffatomen, eine Aryloxygruppe mit 6 bis 10 Kohlenstoffatomen oder eine Gruppe, dargestellt durch die nachstehende Formel (4) ist.

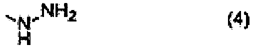

3. Gas-erzeugendes Mittel gemäß Anspruch 1, wobei die Verbindung, dargestellt durch die allgemeine Formel (1), eine Verbindung ist, dargestellt durch die nachstehende Formel (5).

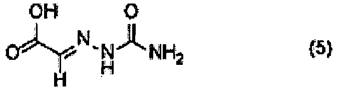

4. Gas-erzeugendes Mittel gemäß Anspruch 1, wobei die Verbindung, dargestellt durch die allgemeine Formel (1), eine Verbindung, dargestellt durch die nachstehende Formel (6), ist.

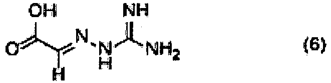

5. Gas-erzeugendes Mittel gemäß Anspruch 1, wobei die Verbindung, dargestellt durch die allgemeine Formel (1), eine Verbindung, dargestellt durch die nachstehende allgemeine Formel (7), ist: 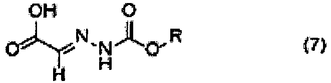 worin R ein Wasserstoffatom, eine Alkylgruppe mit 1 bis 8 Kohlenstoffatomen, eine Allylgruppe mit 3 bis 8 Kohlenstoffatomen, eine Benzylgruppe, eine Alkenylgruppe mit 2 bis 8 Kohlenstoffatomen oder eine Arylgruppe mit 6 bis 10 Kohlenstoffatomen ist.

6. Gas-erzeugendes Mittel gemäß Anspruch 1, wobei die Verbindung, dargestellt durch die allgemeine Formel (1), eine Verbindung, dargestellt durch die nachstehende Formel (8), ist.

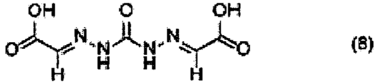

7. Gas-erzeugendes Mittel gemäß einem der Ansprüche 1 bis 6, wobei das Gas-erzeugende Mittel ein Mittel auf Mikrokapsel-Basis ist.

8. Schäumbare Zusammensetzung, umfassend das Gas-erzeugende Mittel gemäß einem der Ansprüche 1 bis 7 und ein zu schäumendes Material.

9. Schäumbare Zusammensetzung gemäß Anspruch 8, wobei das zu schäumende Material ein thermoplastisches Harz und/oder ein Kautschuk ist.

10. Verfahren zur Herstellung eines Schaums, umfassend einen Schritt des Erwärmens der schäumbaren Zusammensetzung gemäß Anspruch 8 oder 9.

11. Schaum, wobei der Schaum durch Schäumen der schäumbaren Zusammensetzung gemäß Anspruch 8 oder 9 erhalten worden ist.

12. Verbindung, dargestellt durch die nachstehende Formel (8).

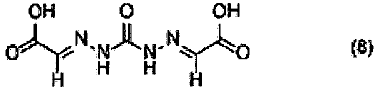

## Revendications

1. Agent générateur de gaz comprenant un composé représenté par la formule générale (1) ci-dessous : 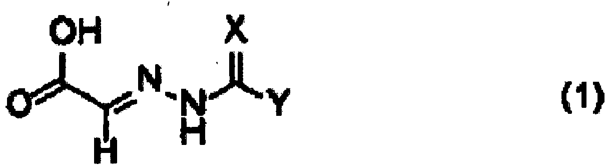 dans laquelle X est un atome d'oxygène ou un groupe imino, et Y est un groupe amino, un groupe hydroxy, un groupe alcoxy ayant 1 à 8 atomes de carbone, un groupe allyloxy ayant 3 à 8 atomes de carbone, un groupe benzyloxy, un groupe alkényloxy ayant 2 à 8 atomes de carbone, un groupe aryloxy ayant 6 à 10 atomes de carbone ou un groupe représenté par la formule (2) ci-dessous.

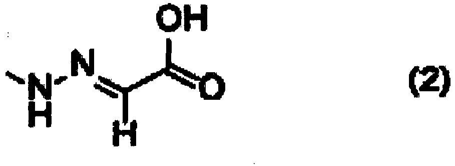

2. Agent générateur de gaz selon la revendication 1, dans lequel le composé représenté par la formule générale (1) comprend une structure dérivée d'un composé (A) représenté par la formule générale (3) ci-dessous et une structure dérivée d'acide glyoxylique (B) : 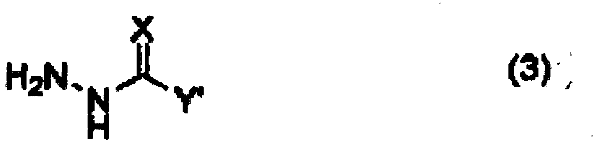 dans laquelle X est un atome d'oxygène ou un groupe imino, et Y' est un groupe amino, un groupe hydroxy, un groupe alcoxy ayant 1 à 8 atomes de carbone, un groupe allyloxy ayant 3 à 8 atomes de carbone, un groupe benzyloxy, un groupe alkényloxy ayant 2 à 8 atomes de carbone, un groupe aryloxy ayant 6 à 10 atomes de carbone ou un groupe représenté par la formule (4) ci-dessous.

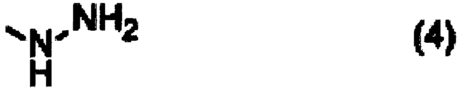

3. Agent générateur de gaz selon la revendication 1, dans lequel le composé représenté par la formule générale (1) est un composé représenté par la formule (5) ci-dessous.

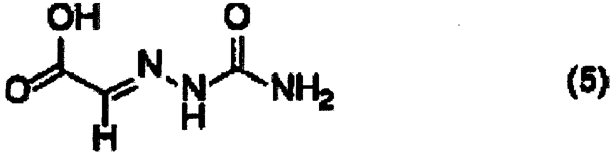

4. Agent générateur de gaz selon la revendication 1, dans lequel le composé représenté par la formule générale (1) est un composé représenté par la formule (6) ci-dessous.

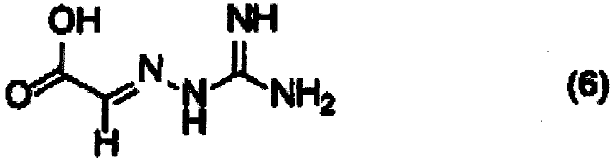

5. Agent générateur de gaz selon la revendication 1, dans lequel le composé représenté par la formule générale (1) est un composé représenté par la formule générale (7) ci-dessous : 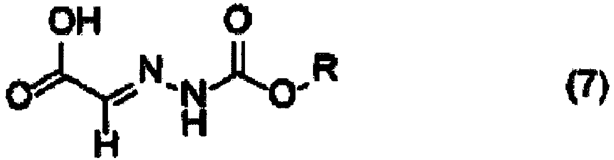 dans laquelle R est un atome d'hydrogène, un groupe alkyle ayant 1 à 8 atomes de carbone, un groupe allyle ayant 3 à 8 atomes de carbone, un groupe benzyle, un groupe alkényle ayant 2 à 8 atomes de carbone ou un groupe aryle ayant 6 à 10 atomes de carbone.

6. Agent générateur de gaz selon la revendication 1, dans lequel le composé représenté par la formule générale (1) est un composé représenté par la formule (8) ci-dessous.

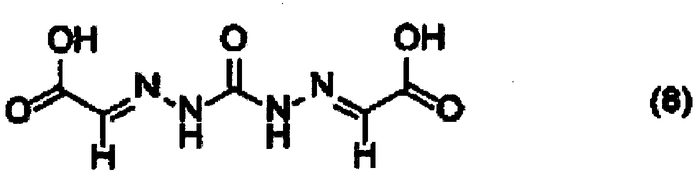

7. Agent générateur de gaz selon l'une quelconque des revendications 1 à 6, dans lequel l'agent générateur de gaz est un agent à base de microcapsule.

8. Composition expansible comprenant l'agent générateur de gaz selon l'une quelconque des revendications 1 à 7 et un matériau devant être expansé.

9. Composition expansible selon la revendication 8, dans laquelle le matériau devant être expansé est une résine thermoplastique et/ou un caoutchouc.

10. Procédé de production d'une mousse comprenant une étape consistant à chauffer la composition expansible selon la revendication 8 ou 9.

11. Mousse, dans laquelle la mousse est obtenue en expansant la composition expansible selon la revendication 8 ou 9.

12. Composé représenté par la formule (8) ci-dessous.
